# EUROPEAN PATENT APPLICATION

(11) **EP 1 849 426 A2**
(43) Date of publication of application: **31.10.2007**
(21) Application number: 07251676.8
(22) Date of filing: 23.04.2007
(51) Int. Cl.: A61B 18/14, A61B 17/32

(54) **Medical instrument having a medical needle-knife**

(30) Priority: 24.04.2006 US 409721
(71) Applicant: ETHICON ENDO-SURGERY, INC., Cincinnati, Ohio 45242 (US)
(72) Inventor: Lu, Ifung, Cincinnati, Ohio 45227 (US); Nobis, Rudolph H., Mason, Ohio 45040 (US)
(74) Representative: Tunstall, Christopher Stephen

(57) **Abstract**

A medical instrument includes a resiliently flexible first elongate member, a resiliently flexible second elongate member, and a medical needle-knife. The first elongate member has a first proximal end portion and a first distal portion. The second elongate member has a second proximal end portion and a second distal portion, wherein the second distal portion is attached to the first distal portion, and wherein the attached first and second distal portions define a distal loop. The medical needle-knife is attached to the distal loop and is insertable within a patient.

## Description

**Field of the Invention**

The present invention is related generally to medical equipment, and more particularly to a medical instrument having a medical needle-knife.

**Background of the Invention**

Endoscopes (including colonoscopes) are known which have an insertion tube which is insertable within a patient. The insertion tube has an articulatable distal end portion controlled by wires running from the distal end portion to control knobs on the handle of the endoscope. A wide angle video camera in the distal end of the insertion tube permits medical observation. A medical needle-knife assembly is part of a known endoscopic system and includes a medical needle-knife attached to a wire with both needle-knife and wire surrounded by a shaft. The shaft is insertable into a working channel of the insertion tube of the endoscope and is translatable to the distal end portion of the endoscope insertion tube. Then, the wire is lengthwise translated to extend the medical needle-knife from the shaft and from the distal end portion of the endoscope insertion tube. Then, in one example, the medical needle-knife is used to provide medical treatment by energizing the wire with energy from a radio-frequency generator. A medical snare assembly is known which includes a wire having a lengthwise translatable first end and having a second end which is fixedly attached to the wire after forming a distal loop snare or which is fixedly attached to a handpiece after forming a distal loop snare.

Still, scientists and engineers continue to seek improved medical instruments having a medical needle-knife.

**Summary of the Invention**

A first expression of an embodiment of the invention is for a medical instrument including a resiliently flexible first elongate member, a resiliently flexible second elongate member, and a medical needle-knife. The first elongate member has a first proximal end portion and a first distal portion. The second elongate member has a second proximal end portion and a second distal portion, wherein the second distal portion is attached to the first distal portion, and wherein the attached first and second distal portions define a distal loop. The medical needle-knife is attached to the distal loop and is insertable within a patient.

A second expression of an embodiment of the invention is for a medical instrument including a resiliently flexible first elongate member, a resiliently flexible second elongate member, a medical needle-knife, and a flexible shaft. The first elongate member has a first proximal end portion and a first distal portion. The second elongate member has a second proximal end portion and a second distal portion, wherein the second distal portion is attached to the first distal portion, and wherein the attached first and second distal portions define a distal loop. The medical needle-knife is attached to the distal loop. The shaft has proximal and distal shaft ends, first and second proximal lumens, and a distal lumen. The distal shaft end is insertable within a patient. The first and second proximal lumens extend from the proximal shaft end toward the distal shaft end. The distal lumen extends from the distal shaft end toward the proximal shaft end and is in communication with each of the first and second proximal lumens. The first elongate member is located in the first proximal lumen, and the second elongate member is located in the second proximal lumen. The medical needle-knife is retractable into the distal lumen and is extendable from the distal lumen.

Several benefits and advantages are obtained from one or more of the expressions of an embodiment of the invention. In one example, an articulatable medical needle-knife is provided wherein distal lengthwise translation of the first proximal end portion with respect to the second proximal end portion articulates the medical needle-knife to a first side, and distal lengthwise translation of the second proximal end portion with respect to the first proximal end portion articulates the medical needle-knife to a second side opposite to the first side. In the same or a different example, the distal loop and the medical needle-knife lie substantially in a plane when the distal loop is in a relaxed state, wherein rotation of the first proximal end portion about its centerline articulates the medical needle-knife out of the plane. In one application, the medical instrument is inserted into a working channel of a flexible insertion tube of an endoscope, wherein the medical needle-knife can be articulated with respect to the insertion tube of the endoscope allowing independent alignment of the wide angle video camera of the endoscope and the medical needle-knife. In a second application, the medical instrument is adapted to be coupled to, and slid along, an exterior rail of a flexible insertion tube of an endoscope allowing independent alignment of the wide angle video camera of the endoscope and the medical needle-knife.

The following is a non-exhaustive list of embodiments of the invention that may be claimed in this application or in subsequently filed divisional applications.

Embodiment 1. A medical instrument comprising: a) a resiliently flexible first elongate member having a first proximal end portion and a first distal portion; b) a resiliently flexible second elongate member having a second proximal end portion and a second distal portion, wherein the second distal portion is attached to the first distal portion, and wherein the attached first and second distal portions define a distal loop; and c) a medical needle-knife attached to the distal loop and insertable within a patient.

Embodiment 2. The medical instrument of embodiment 1, wherein the second distal portion is monolithically attached to the first distal portion.

Embodiment 3. The medical instrument of embodiment 2, wherein the medical needle-knife is monolithically attached to the distal loop.

Embodiment 4. The medical instrument of embodiment 1, wherein the medical needle-knife is a radio-frequency-energized medical needle-knife.

Embodiment 5 The medical instrument of embodiment 4, also including electrical insulation covering at least some of the first distal portion and at least some of the second distal portion.

Embodiment 6. The medical instrument of embodiment l, wherein the medical needle-knife is insertable within a working channel of an endoscope insertion tube.

Embodiment 7. The medical instrument of embodiment 1, wherein the first and second elongate members and the medical needle-knife consist essentially of nitinol wire.

Embodiment 8. The medical instrument of embodiment 7, wherein the distal loop has a relaxed state, wherein the distal loop has substantially a kite shape in the relaxed state, wherein the kite shape has a top, and wherein the medical needle-knife is attached to the distal loop at the top.

Embodiment 9. The medical instrument of embodiment 1, wherein the first proximal end portion is lengthwise translatable with respect to the second proximal end portion, wherein the second proximal end portion is lengthwise translatable with respect to the first proximal end portion, wherein distal lengthwise translation of the first proximal end portion with respect to the second proximal end portion articulates the medical needle-knife to a first side, and wherein distal lengthwise translation of the second proximal end portion with respect to the first proximal end portion articulates the medical needle-knife to a second side opposite to the first side.

Embodiment 10. The medical instrument of embodiment 9, wherein the first proximal end portion has a centerline and is rotatable about the centerline, wherein the distal loop has a relaxed state, wherein the distal loop and the medical needle-knife lie substantially in a plane in the relaxed state, and wherein rotation of the first proximal end portion about the centerline articulates the medical needle-knife out of the plane.

Embodiment 11. A medical instrument comprising: a) a resiliently flexible first elongate member having a first proximal end portion and a first distal portion; b) a resiliently flexible second elongate member having a second proximal end portion and a second distal portion, wherein the second distal portion is attached to the first distal portion, and wherein the attached first and second distal portions define a distal loop; c) a medical needle-knife attached to the distal loop; and d) a flexible shaft having a proximal shaft end, a distal shaft end insertable within a patient, first and second proximal lumens extending from the proximal shaft end toward the distal shaft end, and a distal lumen extending from the distal shaft end toward the proximal shaft end and in communication with each of the first and second proximal lumens, wherein the first elongate member is disposed in the first proximal lumen, wherein the second elongate member is disposed in the second proximal lumen, and wherein the medical needle-knife is retractable into the distal lumen and is extendable from the distal lumen.

Embodiment 12. The medical instrument of embodiment 11, wherein the second distal portion is monolithically attached to the first distal portion.

Embodiment 13. The medical instrument of embodiment 12, wherein the medical needle-knife is monolithically attached to the distal loop.

Embodiment 14. The medical instrument of embodiment 11, wherein the medical needle-knife is a radio-frequency-energized medical needle-knife.

Embodiment 15 The medical instrument of embodiment 14, also including electrical insulation covering at least some of the first distal portion and at least some of the second distal portion.

Embodiment 16. The medical instrument of embodiment 11, wherein the shaft is insertable within a working channel of an endoscope insertion tube.

Embodiment 17. The medical instrument of embodiment 11, wherein the first and second elongate members and the medical needle-knife consist essentially of nitinol wire.

Embodiment 18. The medical instrument of embodiment 17, wherein the distal loop has a relaxed state, wherein the distal loop has substantially a kite shape in the relaxed state, wherein the kite shape has a top, and wherein the medical needle-knife is attached to the distal loop at the top.

Embodiment 19. The medical instrument of embodiment 11, wherein the first proximal end portion is lengthwise translatable with respect to the second proximal end portion, wherein the second proximal end portion is lengthwise translatable with respect to the first proximal end portion, wherein distal lengthwise translation of the first proximal end portion with respect to the second proximal end portion articulates the medical needle-knife to a first side when the medical needle-knife has been extended from the distal lumen, and wherein distal lengthwise translation of the second proximal end portion with respect to the first proximal end portion articulates the medical needle-knife to a second side opposite to the first side when the medical needle-knife has been extended from the distal lumen.

Embodiment 20. The medical instrument of embodiment 19, wherein the first proximal end portion has a centerline and is rotatable about the centerline, wherein the distal loop has a relaxed state, wherein the distal loop and the medical needle-knife lie substantially in a plane in the relaxed state, and wherein rotation of the first proximal end portion about the centerline articulates the medical needle-knife out of the plane when the medical needle-knife has been extended from the distal lumen.

The present invention has, without limitation, application in hand-activated instruments as well as in robotic-assisted instruments.

**Brief Description of the Figures**

FIGURE 1 is a schematic, side elevational, cross sectional view of an embodiment of a medical instrument of the invention showing the distal loop of the medical instrument in a relaxed state;

FIGURE 2 is a cross sectional view of the medical instrument of Figure 1 taken along lines 2-2 in Figure 1;

FIGURE 3 is a view, as in Figure 1, but with the medical instrument of Figure 1 placed within a working channel of an endoscope insertion tube, wherein the medical needle-knife is shown extended from the distal lumen of the shaft and articulated to one side, wherein the electrical insulation of the distal portions of the elongate members, the endoscope handle and operational features (such as insertion tube articulation features and a wide angle video camera) of the endoscope insertion tube have been omitted for clarity; and

FIGURE 4 is a view, as in Figure 2, but showing the medical needle-knife retracted into the distal lumen of the shaft, wherein the electrical insulation of the distal portions of the elongate members have been omitted for clarity.

**Detailed Description of the Invention**

Before explaining the present invention in detail, it should be noted that the invention is not limited in its application or use to the details of construction and arrangement of parts illustrated in the accompanying drawings and description. The illustrative embodiment of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiment of the present invention for the convenience of the reader and are not for the purpose of limiting the invention.

It is understood that any one or more of the following-described expressions of an embodiment, examples, etc. can be combined with any one or more of the other following-described expressions of an embodiment, examples, etc.

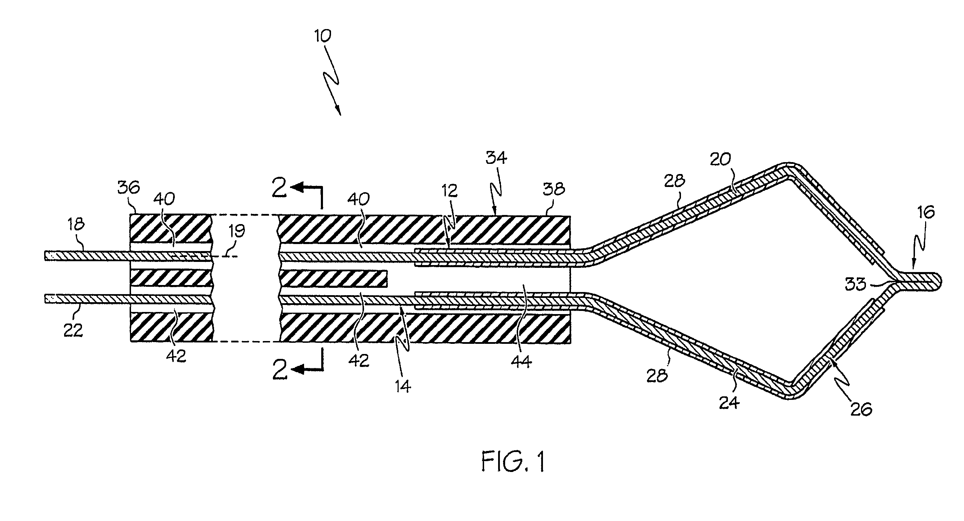

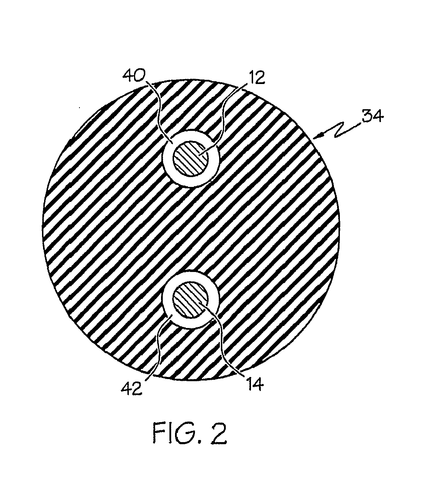

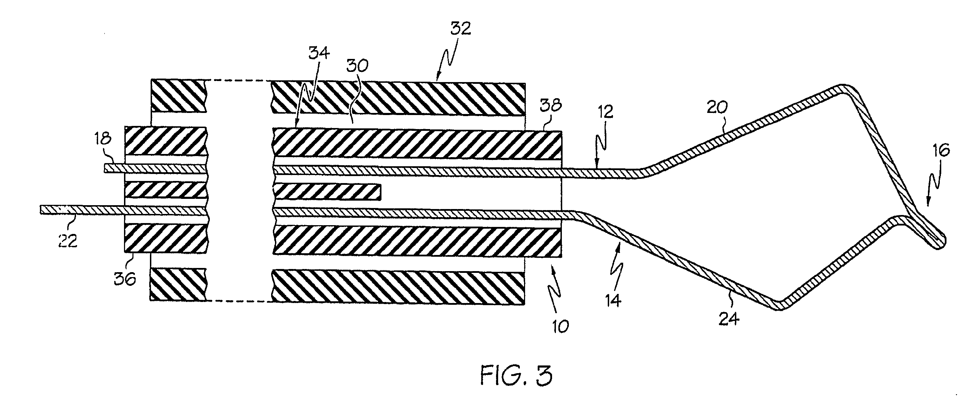

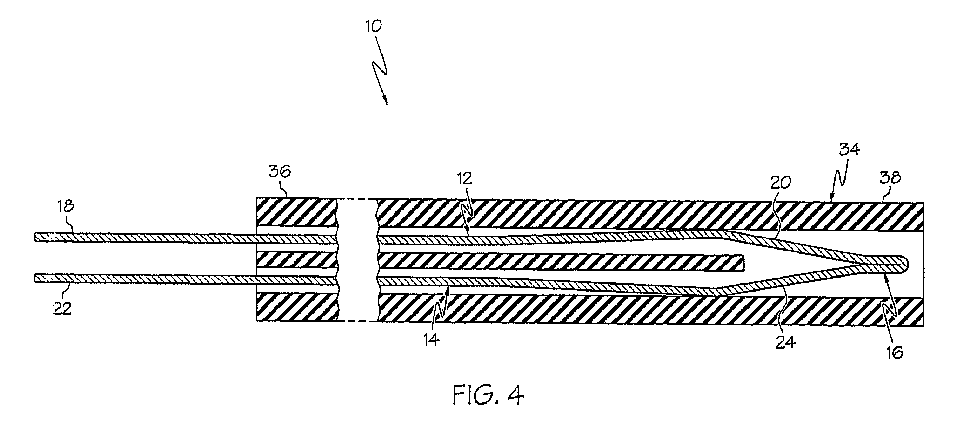

Referring now to the Figures, wherein like numerals represent like elements throughout, Figures 1-4 illustrate an embodiment of the invention. A first expression of the embodiment of Figures 1-4 is for a medical instrument 10 including a resiliently flexible first elongate member 12, a resiliently flexible second elongate member 14, and a medical needle-knife 16. The first elongate member 12 has a first proximal end portion 18 and a first distal portion 20. The second elongate member 14 has a second proximal end portion 22 and a second distal portion 24, wherein the second distal portion 24 is attached to the first distal portion 20, and wherein the attached first and second distal portions 20 and 24 define a distal loop 26. The medical needle-knife 16 is attached to the distal loop 26 and is insertable within a patient.

In one enablement of the first expression of the embodiment of Figures 1-4, the second distal portion 24 is monolithically attached to the first distal portion 20. Thus, in this enablement, the first and second distal portions 20 and 24 are two portions of one continuous piece. In one variation, the medical needle-knife 16 is monolithically attached to the distal loop 26. Thus, in this variation, the medical needle-knife 16 and the distal loop 26 are two portions of one continuous piece. Non-monolithic attachments are left to the artisan. An example, without limitation, of a resiliently flexible member includes a wire. Types of wire include, without limitation, braided wire, monolithic wire, and wire segments lengthwise attached end to end. Other examples of resiliently flexible members and types of wire are left to those skilled in the art.

In one implementation of the first expression of the embodiment of Figures 1-4, the medical needle-knife 16 is a radio-frequency-energized medical needle-knife. Examples of other-energized and non-energized medical needle-knives are left to the artisan. In one variation, the medical instrument 10 also includes electrical insulation 28 covering at least some of the first distal portion 20 and at least some of the second distal portion 24. In one modification, the medical needle-knife 16, or a working portion thereof, is devoid of any electrical insulation, and all other energizable portions of the medical instrument 10 that can contact patient tissue are at all times electrically isolated from patient tissue.

In one application of the first expression of the embodiment of Figures 1-4, the medical needle-knife 16 is insertable within a working channel 30 of an endoscope insertion tube 32.

In a first choice of materials of the first expression of the embodiment of Figures 1-4, the first and second elongate members 12 and 14 and the medical needle-knife 16 consist essentially of nitinol wire. It is noted that nitinol wire is a superelastic wire having shape memory properties wherein the nitinol wire can have a desired shape set into the wire and wherein after flexing the wire, the wire will resiliently return to its set shape, as is known to those skilled in the art. It is noted that the distal loop 26 has a relaxed state (i.e., a state wherein the distal loop 26 it is not subject to a force and wherein the distal loop 26 is not subject to a torque). In one example, the distal loop 26 has substantially a kite shape in the relaxed state (as shown in Figure 1), wherein the kite shape has a top 33, and wherein the medical needle-knife 16 is attached to the distal loop at the top 33. Applicants have found that the kite shape improves the articulation of the medical needle-knife 16. It is noted that a kite shape is a diamond shape having two shorter sides extending from the top (distal) vertex of the diamond shape and having two longer sides extending from the bottom (proximal) vertex of the diamond shape.

In one implementation of the first expression of the embodiment of Figures 1-4, the first proximal end portion 18 is lengthwise translatable with respect to the second proximal end portion 22, and the second proximal end portion 22 is lengthwise translatable with respect to the first proximal end portion 18. Distal lengthwise translation (i.e., lengthwise translation in a distal direction) of the first proximal end portion 18 with respect to the second proximal end portion 22 articulates the medical needle-knife 16 to a first side. Distal lengthwise translation of the second proximal end portion 22 with respect to the first proximal end portion 18 articulates the medical needle-knife 16 to a second side opposite to the first side.

In the same or a different implementation of the first expression of the embodiment of Figures 1-4, the first proximal end portion 18 has a centerline 19 and is rotatable about the centerline 19, the distal loop 26 has a relaxed state, and the distal loop 26 and the medical needle-knife 16 lie substantially in a plane in the relaxed state (e.g., the plane of the paper in Figure 1). Rotation of the first proximal end portion 18 about the centerline 19 articulates the medical needle-knife 16 out of the plane. In one variation, the second proximal end portion is not rotatable about its centerline. In a different variation, the second proximal end portion is rotatable about its centerline. In one modification, both the first and second proximal end portions are rotatable in the same direction about their corresponding centerlines.

A second expression of the embodiment of Figures 1-4 is for a medical instrument 10 including a resiliently flexible first elongate member 12, a resiliently flexible second elongate member 14, a medical needle-knife 16, and a flexible shaft 34. The first elongate member 12 has a first proximal end portion 18 and a first distal portion 20. The second elongate member 14 has a second proximal end portion 22 and a second distal portio 24, wherein the second distal portion 24 is attached to the first distal portion 20, and wherein the attached first and second distal portions 20 and 24 define a distal loop 26. The medical needle-knife 16 is attached to the distal loop 26. The shaft 34 has proximal and distal shaft ends 36 and 38, first and second proximal lumens 40 and 42, and a distal lumen 44. The distal shaft end 38 is insertable within a patient. The first and second proximal lumens 40 and 42 extend from the proximal shaft end 36 toward the distal shaft end 38. The distal lumen 44 extends from the distal shaft end 38 toward the proximal shaft end 36 and is in communication with each of the first and second proximal lumens 40 and 42. The first elongate member 12 is located in the first proximal lumen 40, and the second elongate member 14 is located in the second proximal lumen 42. The medical needle-knife 16 is retractable into the distal lumen 44 and is extendable from the distal lumen 44.

It is noted that the first and second elongate members 12 and 14 are slidingly disposed in the corresponding ones of the first and second proximal and the distal lumens 40, 42 and 44. Simultaneous distal translation of both the first and second proximal end portions 18 and 22 extends the medical snare 26 from the distal lumen 44. Simultaneous proximal translation of both the first and second proximal end portions 18 and 22 retracts the medical snare 26 into the distal lumen 44.

In one enablement of the second expression of the embodiment of Figures 1-4, the second distal portion 24 is monolithically attached to the first distal portion 20. Thus, in this enablement, the first and second distal portions 20 and 24 are two portions of one continuous piece. In one variation, the medical needle-knife 16 is monolithically attached to the distal loop 26. Thus, in this variation, the medical needle-knife 16 and the distal loop 26 are two portions of one continuous piece. Non-monolithic attachments are left to the artisan. An example, without limitation, of a resiliently flexible member includes a wire. Types of wire include, without limitation, braided wire, monolithic wire, and wire segments lengthwise attached end to end. Other examples of resiliently flexible members and types of wire are left to those skilled in the art.

In one implementation of the second expression of the embodiment of Figures 1-4, the medical needle-knife 16 is a radio-frequency-energized medical needle-knife. Examples of other-energized and non-energized medical needle-knives are left to the artisan. In one variation, the medical instrument 10 also includes electrical insulation 28 covering at least some of the first distal portion 20 and at least some of the second distal portion 24. In one modification, the medical needle-knife 16, or a working portion thereof, is devoid of any electrical insulation, and all other energizable portions of the medical instrument 10 that can contact patient tissue are at all times electrically isolated from patient tissue.

In one application of the second expression of the embodiment of Figures 1-4, the shaft 34 is insertable within a working channel 30 of an endoscope insertion tube 32. In another application, not shown, the shaft has a rail-coupling feature adapted to be coupled to, and slid along, an exterior rail of a flexible insertion tube of an endoscope. Other applications, including non-endoscope use of the medical instrument 10, are left to those skilled in the art.

In a first choice of materials of the second expression of the embodiment of Figures 1-4, the shaft 34 comprises an elastomer, and the first and second elongate members 12 and 14 and the medical needle-knife 16 consist essentially of nitinol wire. It is noted that nitinol wire is a superelastic wire having shape memory properties wherein the nitinol wire can have a desired shape set into the wire and wherein after flexing the wire, the wire will resiliently return to its set shape, as is known to those skilled in the art. It is noted that the distal loop 26 has a relaxed state (i.e., a state wherein the distal loop 26 it is not subject to a force and wherein the distal loop 26 is not subject to a torque). In one example, the distal loop 26 has substantially a kite shape in the relaxed state (as shown in Figure 1), wherein the kite shape has a top 33, and wherein the medical needle-knife 16 is attached to the distal loop at the top 33. Applicants have found that the kite shape improves the articulation of the medical needle-knife 16. It is noted that a kite shape is a diamond shape having two shorter sides extending from the top (distal) vertex of the diamond shape and having two longer sides extending from the bottom (proximal) vertex of the diamond shape.

In one implementation of the second expression of the embodiment of Figures 1-4, the first proximal end portion 18 is lengthwise translatable with respect to the second proximal end portion 22, and the second proximal end portion 22 is lengthwise translatable with respect to the first proximal end portion 18. Distal lengthwise translation (i.e., lengthwise translation in a distal direction) of the first proximal end portion 18 with respect to the second proximal end portion 22 articulates the medical needle-knife 16 to a first side when the medical needle-knife 16 has been extended from the distal lumen 44. Distal lengthwise translation of the second proximal end portion 22 with respect to the first proximal end portion 18 articulates the medical needle-knife 16 to a second side opposite to the first side when the medical needle-knife 16 has been extended from the distal lumen 44.

In the same or a different implementation of the second expression of the embodiment of Figures 1-4, the first proximal end portion 18 has a centerline 19 and is rotatable about the centerline 19, the distal loop 26 has a relaxed state, and the distal loop 26 and the medical needle-knife 16 lie substantially in a plane in the relaxed state (e.g., the plane of the paper in Figure 1). Rotation of the first proximal end portion 18 about the centerline 19 articulates the medical needle-knife 16 out of the plane when the medical needle-knife 16 has been extended from the distal lumen 44. In one variation, the second proximal end portion is not rotatable about its centerline. In a different variation, the second proximal end portion is rotatable about its centerline. In one modification, both the first and second proximal end portions are rotatable in the same direction about their corresponding centerlines.

In one employment of the first and/or second expression of the embodiment of Figures 1-4, the medical instrument 10 includes a handpiece, not shown. In one example, the handpiece includes a joystick-type handle operatively connected to the first proximal end portion 18 of the first elongate member 12 and to the second proximal end portion 22 of the second elongate member 14, wherein moving the joystick handle to one side articulates the medical needle-knife 16 to one side and moving the joystick handle to the other side articulates the medical needle-knife 16 to the other side, wherein the first proximal end portion 18 of the first elongate member 12 has a square cross section, and wherein rotation of a ring on the handpiece rotates the first proximal end portion 18 inside the handpiece through a gear arrangement. In another example, not shown, the handpiece has one stationary finger ring for support and has first and second slidable finger rings connected to a corresponding one of the first and second proximal end portions 18 and 22 for lengthwise translation thereof, wherein the first proximal end portion 18 of the first elongate member 12 has a square cross section, and wherein rotation of a ring on the handpiece rotates the first proximal end portion 18 inside the handpiece through a gear arrangement. Other examples of handpieces and robotic operation of the medical instrument 10 are left to those skilled in the art. In a different employment, a user manually translates and/or rotates the first and/or second proximal end portions 18 and 22 of the first and/or second elongate members 12 and 14 to articulate the medical needle-knife 16.

In one procedure involving the second expression of the embodiment of Figures 1-4, both the first and second proximal end portions 18 and 22 are lengthwise translated to retract the medical needle-knife 16 within the distal lumen 44 before the shaft 34 is inserted within a patient (such as before the shaft 34 is inserted within a working channel 30 of an endoscope insertion tube 32 which has been inserted within a patient). When the distal shaft end 38 has been positioned proximate the target tissue requiring medical treatment, both the first and second proximal end portions 18 and 22 are lengthwise translated to extend the medical needle-knife 16 from the distal lumen 44 (and from the endoscope insertion tube 32, if present). Thereafter, the medical needle-knife 16 is articulated to a desired orientation for medical treatment.

Several benefits and advantages are obtained from one or more of the expressions of an embodiment of the invention. In one example, an articulatable medical needle-knife is provided wherein distal lengthwise translation of the first proximal end portion with respect to the second proximal end portion articulates the medical needle-knife to a first side, and distal lengthwise translation of the second proximal end portion with respect to the first proximal end portion articulates the medical needle-knife to a second side opposite to the first side. In the same or a different example, the distal loop and the medical needle-knife lie substantially in a plane when the distal loop is in a relaxed state, wherein rotation of the first proximal end portion about its centerline articulates the medical needle-knife out of the plane. In one application, the medical instrument is inserted into a working channel of a flexible insertion tube of an endoscope, wherein the medical needle-knife can be articulated with respect to the insertion tube of the endoscope allowing independent alignment of the wide angle video camera of the endoscope and the medical needle-knife. In a second application, the medical instrument is adapted to be coupled to, and slid along, an exterior rail of a flexible insertion tube of an endoscope allowing independent alignment of the wide angle video camera of the endoscope and the medical needle-knife.

While the present invention has been illustrated by a description of several expressions of an embodiment, it is not the intention of the applicants to restrict or limit the spirit and scope of the appended claims to such detail. Numerous other variations, changes, and substitutions will occur to those skilled in the art without departing from the scope of the invention. For instance, the medical instrument of the invention has application in robotic assisted surgery taking into account the obvious modifications of such systems, components and methods to be compatible with such a robotic system. It will be understood that the foregoing description is provided by way of example, and that other modifications may occur to those skilled in the art without departing from the scope and spirit of the appended Claims.

## Claims

1. A medical instrument comprising:
a) a resiliently flexible first elongate member having a first proximal end portion and a first distal portion;
b) a resiliently flexible second elongate member having a second proximal end portion and a second distal portion, wherein the second distal portion is attached to the first distal portion, and wherein the attached first and second distal portions define a distal loop; and
c) a medical needle-knife attached to the distal loop and insertable within a patient.

2. The medical instrument of claim 1, wherein the second distal portion is monolithically attached to the first distal portion.

3. The medical instrument of claim 2, wherein the medical needle-knife is monolithically attached to the distal loop.

4. The medical instrument of claim 1, wherein the medical needle-knife is a radio-frequency-energized medical needle-knife.

5. The medical instrument of claim 4, also including electrical insulation covering at least some of the first distal portion and at least some of the second distal portion.

6. The medical instrument of claim 1, wherein the medical needle-knife is insertable within a working channel of an endoscope insertion tube.

7. The medical instrument of claim 1, wherein the first and second elongate members and the medical needle-knife consist essentially of nitinol wire.

8. The medical instrument of claim 7, wherein the distal loop has a relaxed state, wherein the distal loop has substantially a kite shape in the relaxed state, wherein the kite shape has a top, and wherein the medical needle-knife is attached to the distal loop at the top.

9. The medical instrument of claim 1, wherein the first proximal end portion is lengthwise translatable with respect to the second proximal end portion, wherein the second proximal end portion is lengthwise translatable with respect to the first proximal end portion, wherein distal lengthwise translation of the first proximal end portion with respect to the second proximal end portion articulates the medical needle-knife to a first side, and wherein distal lengthwise translation of the second proximal end portion with respect to the first proximal end portion articulates the medical needle-knife to a second side opposite to the first side.

10. The medical instrument of claim 9, wherein the first proximal end portion has a centerline and is rotatable about the centerline, wherein the distal loop has a relaxed state, wherein the distal loop and the medical needle-knife lie substantially in a plane in the relaxed state, and wherein rotation of the first proximal end portion about the centerline articulates the medical needle-knife out of the plane.

11. A medical instrument comprising:
a) a resiliently flexible first elongate member having a first proximal end portion and a first distal portion;
b) a resiliently flexible second elongate member having a second proximal end portion and a second distal portion, wherein the second distal portion is attached to the first distal portion, and wherein the attached first and second distal portions define a distal loop;
c) a medical needle-knife attached to the distal loop; and
d) a flexible shaft having a proximal shaft end, a distal shaft end insertable within a patient, first and second proximal lumens extending from the proximal shaft end toward the distal shaft end, and a distal lumen extending from the distal shaft end toward the proximal shaft end and in communication with each of the first and second proximal lumens, wherein the first elongate member is disposed in the first proximal lumen, wherein the second elongate member is disposed in the second proximal lumen, and wherein the medical needle-knife is retractable into the distal lumen and is extendable from the distal lumen.
